# EUROPEAN PATENT SPECIFICATION

(11) **EP 3 137 121 B1**
(45) Date of publication and mention of the grant of the patent: **11.12.2024**
(21) Application number: 15785448.0
(22) Date of filing: 28.04.2015
(51) Int. Cl.: B43K 15/00, A61L 2/10

(54) **DECONTAMINATION METHOD AND APPARATUS**
DEKONTAMINATIONSVERFAHREN UND VORRICHTUNG
PROCÉDÉ ET APPAREIL DE DÉCONTAMINATION

(30) Priority: 28.04.2014 US 201461985246 P; 28.04.2014 US 201461985234 P
(43) Date of publication of application: 08.03.2017
(73) Proprietor: Diversey, Inc., Fort Mill, SC 29708 (US)
(72) Inventor: DAYTON, Roderick, M., Strongsville, OH 44136 (US)
(74) Representative: LKGlobal UK Ltd.
(86) International application number: PCT/US2015/027976
(87) International publication number: WO 2015/168111

(56) References cited:
- EP-A1- 0 959 696
- EP-B1- 0 959 696
- CN-A- 103 550 801
- KR-A- 20110 132 692
- KR-A- 20120 052 521
- RU-U1- 16 257
- US-A- 5 446 289
- US-A- 6 165 526
- US-A1- 2010 018 240
- US-A1- 2010 018 240
- US-A1- 2012 261 593
- US-A1- 2014 060 094
- US-B2- 7 507 369
- US-B2- 8 193 515

## Description

### BACKGROUND OF THE INVENTION

### 1. Field of the Invention

This application relates generally to a method and apparatus for reducing contagions on an object and, more specifically, to a method and apparatus for suitably exposing an object to be used in a substantially-sterile environment to a disinfectant, which can optionally be a sterilizing agent.

### 2. Description of Related Art

Sterile, or at least substantially-sterile environments are common in the medical field for treating patients with minimal risk of infection. To avoid exposing patients in such environments to infectious organisms medical personnel working therein are required to take precautionary measures. All personnel are required to wash thoroughly before entering the environment, and wear items of clothing such as surgical scrubs that have been decontaminated.

Other objects such as medical equipment can also be contaminated with infectious organisms, and can pose a threat to introduce such organisms into the sterile environment. Bedding, medical devices, and virtually all other objects brought into a sterile environment must undergo sterilization procedures to minimize the risk of infection to patients. More recently, portable electronic devices such as tablet computers, for example, have become useful within sterile environments such as an operating room during a surgical procedure. Pulse oximeters, keyboards, and any other object that is often touched by hospital personnel or patients can also provide a means for transmitting infectious organisms when not properly and consistently decontaminated.

The wide array of electronic devices such as tablet computers and notebook computers, and medical devices, for example, that require decontamination pose additional problems when being considered for use in a medical environment. Their cases include apertures, seams, internal compartments and a variety of other structures where infectious organisms can hide from a disinfectant or sterilizing agent, which is often topically applied as part of a decontamination process. Thus, even when exposed to such a decontaminant or sterilizing agent, infectious organisms hidden at such locations on an electronic device can be unknowingly transported into the sterile environment.

Conventionally writing instruments such as ink pens include a cylindrical housing defining an interior compartment. An ink reservoir disposed within the interior compartment stores ink that is to be dispensed onto a medium as the user is writing. A writing tip such as a ball-point, for example, is coupled in fluid communication to the ink reservoir and extends from the ink reservoir through an aperture located at a terminal end of the housing.

Retractable pens include an adjustable writing tip. The adjustable writing can be adjusted inwardly to a stowed position, where the writing tip is retracted through the aperture at the terminal end of the housing into the interior chamber. When the retractable pen is to be used, the writing tip can be adjusted outwardly through the aperture to a writing position where the writing tip extends beyond the housing, thereby exposing the ball point or other ink-dispensing surface to make contact with the medium. A button located at an end of the housing opposite the aperture can be pushed into the housing through another aperture to urge the writing tip toward the writing position, and pushed again to retract the writing tip to the stowed position. Although the inside diameter of each aperture is within a close tolerance of the outside diameter of the respective writing tip and button, there remains a space separating those objects from the interior periphery of their respective apertures through which a contaminant can enter the interior chamber of the pen.

In medical settings such as hospitals many patients may be treated by physicians, nurses and other caregivers who travel from room to room. In each patient's room the caregiver's hands are likely to make physical contact with the patient or other objects during the course of treating the patient. This physical contact can expose the caregiver's hands to germs and other possible contaminants that, unless the proper disinfection techniques are followed, could be transferred to another patient in a different room visited by the caregiver. These contaminants can also be spread to objects held by the caregiver, such as pens or other writing instruments, for example.
Prior art document CN 103550801 discloses an apparatus for disinfecting objects of various shapes and sizes based on UV radiation. Another example is shown in EP 0 959 696.

### BRIEF SUMMARY OF THE INVENTION

Accordingly, there is a need in the art for a method and apparatus for sterilizing the variety of commonly used devices in the medical field regardless of the particular shape of the object.

There is also a need in the art for an ink-based writing instrument with an interior chamber that is substantially sealed from an ambient environment to interfere with the intrusion of contaminants into the interior chamber. There is also a need in the art for a disinfection station that exposes writing instruments to a disinfecting agent when not in use and stores disinfected writing instruments in a disinfected environment.

Therefore, the invention relates to an apparatus for disinfecting objects of various shapes and sizes by placing the object in a housing enclosing a disinfecting chamber as specified in claim 1.

According to another aspect, the subject application involves a method for using the above-described apparatus for disinfecting commonly used devices and objects, particularly in the medical field.

The above summary presents a simplified summary in order to provide a basic understanding of some aspects of the systems and/or methods discussed herein. This summary is not an extensive overview of the systems and/or methods discussed herein. It is not intended to identify key/critical elements or to delineate the scope of such systems and/or methods. Its sole purpose is to present some concepts in a simplified form as a prelude to the more detailed description that is presented later.

### BRIEF DESCRIPTION OF SEVERAL VIEWS OF THE DRAWING

The invention may take physical form in certain parts and arrangement of parts, embodiments of which will be described in detail in this specification and illustrated in the accompanying drawings which form a part hereof and wherein:
FIG. 1 is a perspective view of an illustrative variant of a retractable pen with a writing tip in a stowed position where the writing tip is at least partially recessed within a housing of the pen, wherein adjustment of the writing tip's position is achieved by actuation of a push button;
FIG. 2 is another perspective view of the retractable pen shown in FIG. 1 with the writing tip adjusted outwardly, to a writing position where the writing tip extends beyond the housing of the pen;
FIG. 3 is a partial sectional view of the pen with the writing tip adjusted to the stowed position taken along line 3-3 in FIG. 1, showing a skirt sealing an aperture through which the writing tip is adjusted to interfere with the intrusion of a contaminant into an interior compartment of the pen;
FIG. 4 is a partial sectional view of the pen with the writing tip adjusted to the writing position taken along line 4-4 in FIG. 2, showing a skirt sealing an aperture through which the writing tip is adjusted to interfere with the intrusion of a contaminant into an interior compartment of the pen;
FIG. 5 is a perspective view of an illustrative variant of a retractable pen with a writing tip in a stowed position, wherein adjustment of the writing tip's position is achieved by pivotally adjusting a first housing segment relative to another housing segment;
FIG. 6 is another perspective view of the retractable pen shown in FIG. 5 with the writing tip adjusted outwardly, in a writing position where the writing tip extends beyond the housing of the pen;
FIG. 7 is a partially cutaway side view of a decontamination station in which writing instruments are to be deposited and exposed to a decontaminating agent;
FIG. 8 is a side view of a variant of a decontamination region that can be disposed within the decontamination station shown in FIG. 7 to at least partially decontaminate a pen or other writing instrument;
FIG. 9 is a sectional view of an the decontamination region taken along line 9-9 in FIG. 8, showing a variant of a bristled surface that contributes to rotation of a pen traveling through the decontamination region;
FIG. 10 is a side view of another variant of a decontamination region that can be disposed within the decontamination station shown in FIG. 7;
FIG. 11 is a sectional view of an the decontamination region taken along line 11-11 in FIG. 10, showing a variant of angled ribs that contribute to rotation of a pen traveling through the decontamination region;
FIG. 12 shows a cross section of an illustrative variant of the present disclosure; and
FIG. 13 shows a top view of an illustrative embodiment of the present invention.

### DETAILED DESCRIPTION OF THE INVENTION

Certain terminology is used herein for convenience only and is not to be taken as a limitation on the present invention. Relative language used herein is best understood with reference to the drawings, in which like numerals are used to identify like or similar items. Further, in the drawings, certain features may be shown in somewhat schematic form.

It is also to be noted that the phrase "at least one of', if used herein, followed by a plurality of members herein means one of the members, or a combination of more than one of the members. For example, the phrase "at least one of a first widget and a second widget" means in the present application: the first widget, the second widget, or the first widget and the second widget. Likewise, "at least one of a first widget, a second widget and a third widget" means in the present application: the first widget, the second widget, the third widget, the first widget and the second widget, the first widget and the third widget, the second widget and the third widget, or the first widget and the second widget and the third widget.

The disinfecting process performed by the apparatus and methods described herein is performed by a disinfecting apparatus on demand, as materially-disinfected substantially-sterilized objects are needed in an application, for example, a medical application. Rendering the object "materially disinfected" does not necessarily require the object to be 100% sterile, free of any and all living organisms. Instead, to be "materially disinfected", the living contagions on the object are exposed to UVC light. Although this exposure may not kill the contagions, the exposed contagions are unable to replicate as a result of the exposure to UVC light, thus promoting a lower level of replicating living contagions on the object after performance of the sterilization process than existed on the object prior to performance of the sterilization process. According to other aspects, the object is required to possess a lower level of living or otherwise biologically-active contagions than a threshold quantity permitted under U.S. Food and Drug Administration requirements on objects dedicated for use in a sterile field such as in an operating room during a surgical procedure. According to other aspects, the sterilization process kills or otherwise eliminates at least 99% of all living or otherwise biologically-active contagions present on the object immediately prior to performance of the sterilization process. According to yet other aspects, achieving high-level disinfection of an object utilizing the disinfecting apparatus can involve deactivation of a suitable portion of the biologically-active contagions to achieve at least a 1 log₁₀ reduction of such contagions on the object that remain infectious (i.e., no more than 1/10^{th} of the biologically-active contagions on the object remain active or infectious at a time when the decontamination process is completed). According to yet other aspects, achieving a low to intermediate-level of disinfection of an object utilizing the disinfecting apparatus can involve deactivation of a suitable portion of the biologically-active contagions to achieve at least a 3 log₁₀ reduction (i.e., 1/1,000^{th}) 99.9% of such contagions on the object. According to yet other aspects, achieving high-level disinfection of an object utilizing disinfecting apparatus can involve deactivation of a suitable portion of the biologically-active contagions to achieve at least a 6 log₁₀ reduction (i.e., 1/1,000,000^{th}) of such contagions on the object. Yet other aspects requiring sterilization of the object can result in a complete and total absence of viable organisms on the object at a time when the decontamination process is completed.

Thus, although referred to as a "disinfecting apparatus" herein for convenience, it is to be understood that the disinfecting apparatus subjects objects to a decontamination process that at least disinfects, and optionally sterilizes, the objects by exposing the objects to a disinfectant, interchangeably referred to herein as a sterilizing agent, to deactivate (e.g., kill or otherwise render no longer infectious) a portion of a biologically-active contaminate present on the objects. Once the decontamination process is complete, the objects are suitable for use in a sterile field such as an operating room during a surgical procedure or other healthcare-related practice.

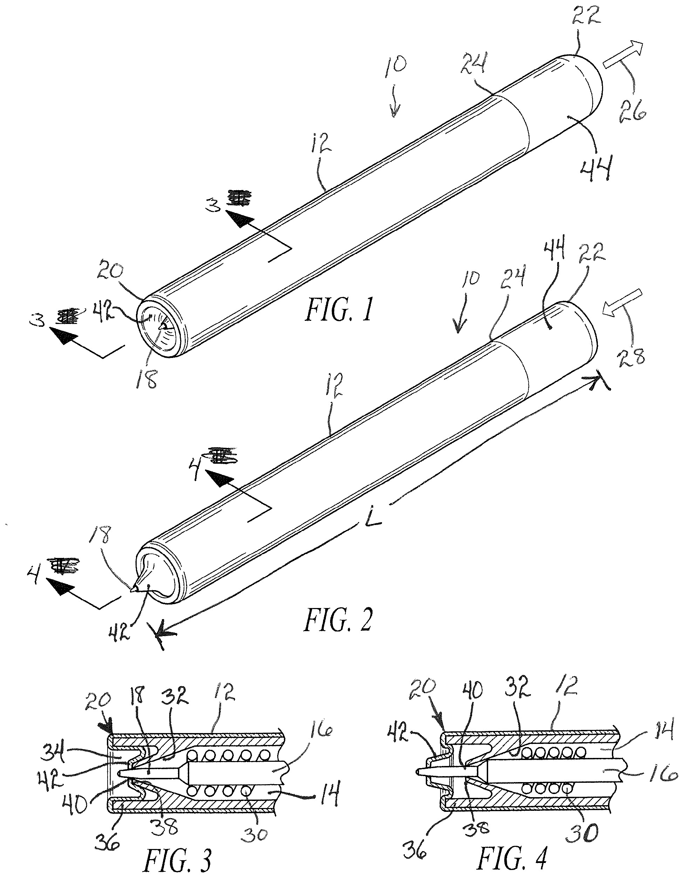

An illustrative variant of writing instrument in the form of a retractable pen 10 is shown in FIGs. 1 and 2. As shown in FIG. 1, the pen 10 is equipped with a writing tip 18 in a stowed position, where the writing tip 18 is at least partially recessed within a first end 20 of a housing 12. Adjustment of the writing tip 18 between the stowed position shown in FIG. 1 and the writing position shown in FIG. 2 is achieved in response to actuation of a push button 22 provided adjacent to a second end 24 of the housing 12. While the writing tip 18 is in the stowed position, the push button 22 is fully extended in an axial direction away from the second end 24 of the housing 24 generally indicated by arrow 26. With the writing tip 18 in the writing position shown in FIG. 2, the push button 22 can be maintained in a relatively inward position in the opposite axial direction indicated generally by arrow 28. Adjustment of the writing tip 18 from the writing position to the stowed position, and vice versa, can be brought about in response to a pushing of the push button 22 in the direction indicated by arrow 28 followed by a release of the push button 22. Although the overall length of the pen 10 can be any desired length, variants of the pen 10 have an overall length L in FIG. 2 from a surface of the push button 22 furthest away from the second end 24 of the housing to the apex of the writing tip 18 of at least about three (3 in.) inches, and optionally less than about 15.24 cm (6 in.). Specific embodiments of the pen 10 have an overall length L that is between about 10.16 cm (4 in.) and about 12.7 cm (5 in.).

An exterior surface of the housing 12 can be generally-cylindrical in shape, or can include a plurality of axially aligned planar surfaces that collectively form an arcuate shape. The housing 12 defines an interior compartment 14, shown in FIGs 3 and 4, in which an ink cartridge 16 is disposed. The ink cartridge 16 is operatively connected in fluid communication with the writing tip 18 to supply the writing tip 18 with ink that is discharged from the writing tip 18 onto a surface, such as paper for example, on which a user of the pen 10 is writing.

A helical spring 30 is provided within the interior compartment 14 to urge the writing tip 18 and, optionally, also the ink cartridge 16 and/or push button 22, in the axial direction indicated generally by arrow 26 in FIG. 1. Thus, the spring 30 urges the writing tip 18 generally toward the stowed position and is compressed against a narrowing portion 32 of the housing 12 defining a portion the interior compartment 14 when the writing tip 18 is adjusted to the writing position.

Shown clearly in FIG. 3, the first end 20 of the housing 12 defines a recess 34 in which at least a portion of the writing tip 18 is disposed in the stowed position. A generally-cylindrical external wall 36, which can optionally be integrally formed as part of a monolithic unit with another portion of the housing 12, forms an arcuate perimeter of the recess 34. A proximate partition 38, shown best in FIG. 4, disposed between the recess 34 and the interior compartment 14 defines an aperture 40 through which the writing tip 18 extends from the interior compartment 14 into the recess 34. The partition 38 can optionally be integrally formed as part of a monolithic unit (e.g., molded, cast, etc...) from the same material as an external surface of the housing 12. Use of a rigid material with suitable strength such as a plastic, metal, other material or combination thereof, offers the writing tip 18 lateral support to remain substantially stationary even while in the writing position and being used by a user who is writing with the pen 10.

To interfere with the entry of contaminants into the interior compartment 14 from the ambient environment of the pen 10, a flexible skirt 42 extends between the perimeter wall 36 and the writing tip 18. The skirt 42 can optionally extend from an external periphery of the wall 36, a terminal surface of the wall 36 in the axial direction that forms an end of the housing 12, or from an internal periphery of the wall 36. Regardless of the surface from which the skirt 42 extends toward the writing tip 18, the externally-exposed surface of the skirt 42 and any portion of the internal periphery of the wall 36 is exposed to light directed into the recess 34 from beyond the first end 20. In other words, the skirt 42 and any exposed portion of the internal periphery of the wall 36 are visible when looking axially into the recess 34 formed at the first end 20, and can optionally form a continuous surface free of hidden surfaces that could be shielded from light aimed into the recess 34 by other portions of the pen 10. For such variants, contaminants such as parasites or other infectious organisms on the skirt 42 and/or interior periphery of the wall 36 can be deactivated or otherwise neutralized by a decontamination agent such as UVC light impart into the recess 34 from a location located axially beyond the first end 20 of the pen 10.

The skirt 42 can be made from any material that is flexible enough to extend with the writing tip 18 to the writing position and be retracted into the recess 34 when the writing tip 18 is adjusted to the stowed position, while maintaining a reasonable seal that interferes with, and optionally prevents, the entry of contaminants into the interior compartment 14 through the recess 34 and aperture 40. As can be seen in FIGs. 1 and 3, while the writing tip 18 is in the stowed position, the skirt 42 is received, along with the writing tip 18, in the recess 34. When the writing tip 18 is extended to the writing position as shown in FIGs. 2 and 4, the skirt 42 can optionally extend outwardly, beyond the recess 34. According to alternate variants, however, the skirt 42 can coupled to a portion of the writing tip 18 such that the skirt 42 remains within the perimeter of the wall 36, and accordingly within the recess 34, even when the writing tip 18 is adjusted to the writing position.

According to yet other variant, the skirt 42 can be formed as a rubber gasket disposed about an interior periphery of the aperture 40 to substantially seal the entry into the interior compartment 14 from the first end 20. The gasket can press tightly against the outside diameter of the writing tip 18 where the writing tip 18 extends through the aperture 40, and maintains this contact against the writing tip 18 as it travels axially through the gasket between the writing and stowed positions. Again, the exposed surfaces of such a gasket and interior periphery of the perimeter wall 36 are not shaded or otherwise shielded from a decontamination agent directed into the recess 34 from beyond the first end 20 of the housing 12.

Similarly, the push button 22 adjacent to the second end 24 of the housing 12 can be concealed by a flexible sheath 44. The sheath 44 can be formed from an elastomeric material to form a cap over the push button 22, yet allow actuation of the push button 22 in response to a force applied to the externally-exposed surface of the sheath 44. The sheath 44 can also be coupled to perimeter of the housing 12 adjacent to the second end 24, and a seal can be applied where the sheath 44 meets the second end 24 of the housing 12. The sheath 44 conceals apertures formed between interworking components of the push button 22 assembly through which contaminants could otherwise potentially enter an interior portion of the pen 10. Additionally, the sheath 44 has a substantially-cylindrical, and smooth externally-exposed surface, allowing for effective decontamination of that externally-exposed surface in response to exposure to a decontamination agent such as UVC light.

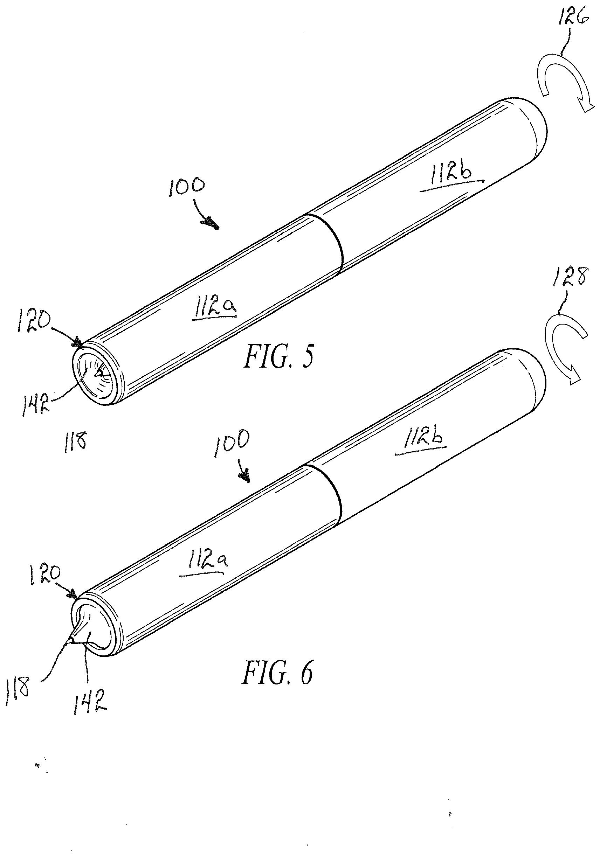

Although the variant of the writing instrument described with reference to FIGs. 1-4 is a push-button ink pen 10, alternate variants of the writing instrument include a pen 100 shown in FIGs. 5 and 6. Such variants include a first end 120 configured to be analogous to the first end 20 of the variants described above. A writing tip 118 is adjustable between a stowed position (FIG. 5) and a writing position (FIG. 6), and a skirt 142 interferes with entry of contaminants into an interior compartment of the pen 100. However, adjustment of the writing tip's position is achieved by rotating a first housing portion 112a relative to a second housing portion 112b in the directions indicated by arrows 126, 128. A seal can be provided at the intersection of the housing portions 112a, 112b to interfere with the entry of contaminants into the interior compartment of the pen 100 and allow for effective decontamination of the pen 100 through exposure of the pen 100 to a decontamination agent that requires direct exposure of the surfaces to be decontaminated to the decontamination agent such as UVC light.

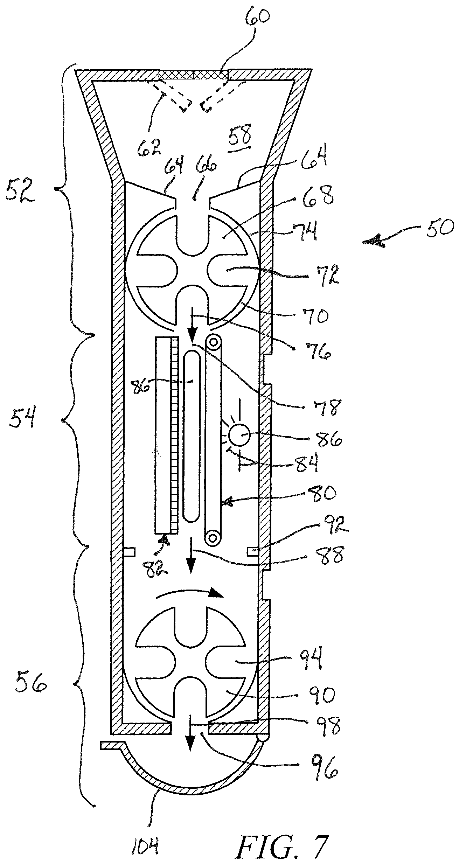

A decontamination station 50 such as that shown in FIG. 7 can be hung on a wall or otherwise placed at a convenient location in a healthcare facility, for example, to decontaminate, and optionally store, the pens 10, 100 described above. A plurality of such decontamination stations 50, each compatible with the pens 10, 100, can be located at various locations throughout the healthcare facility to facilitate ready access to decontaminated pens 10, 100.

The decontamination station 50 of FIG. 7 includes a feed region 52, a decontamination region 54 and a distribution region 56. The feed region 52 includes a hopper 58 into which pens 10, 100 can be deposited through an entry aperture 60. The entry aperture 60 can include one or a plurality of adjustable doors that can be temporarily pushed downward, into the hopper 58 as shown in broken lines 62 to allow a pen 10, 100 to be dropped, or otherwise permit the introduction of a pen 10, 100 into the decontamination station 50. The door(s) can be spring biased, and returned to the closed position once the force pushing the door(s) downward is removed and the pen 10, 100 inserted reaches the hopper 58. According to alternate variants, the entry aperture 60 can include opposing sets of bristles, an elastomeric rubber flap, or other selective entry device that interferes with light or other decontamination agent exiting the decontamination station 50 when closed, but allows the insertion of the pens 10, 100.

Although the decontamination station 50 can be compatible with a plurality of different pens 10, 100, for the sake of brevity and clarity a variant of the decontamination station 50 will be described with reference to push-button embodiment of the pen 10.

The hopper 58 includes tapered walls 64 or other directional structures that funnel pens 10 toward an entrance 66 to a feeding cylinder 68. The pen 10 rolls along the tapered walls 64 until it reaches the entrance, at which time the pen 10 falls into the entrance 66 and reaches the feeding cylinder 68. The pen 10 will rest on an arcuate, outer surface 70 of the feeding cylinder 68 in the entrance 66 while the feeding cylinder 68 rotates. When the feeding cylinder 68 rotates to an extent that a generally U-shaped receptacle 72 formed in the feeding cylinder 68 becomes aligned with the entrance 66, the pen 10 resting therein falls into the receptacle 72. The depth and shape of the receptacle 72 is sufficient to allow the pen 10 therein to be rotated with the feeding cylinder 68 toward an entrance to the decontamination region 54 without striking any surrounding walls 74.

When the pen 10 in the receptacle 72 becomes aligned with an entrance to the decontamination region 54, the pen 10 falls under the force of gravity in the direction of arrow 76 into a space 78 between a moving conveyor 80 and a stationary object 82. While within the space 78, the pen 10 is exposed to a decontamination agent such as UVC light 84 emitted by at least one, and optionally a plurality of UVC bulbs 86 arranged adjacent to the space 78. The conveyor 80 is operable to control movement of the pen 10 through the space 78 to ensure sufficient exposure to the decontamination agent to achieve the desired level of decontamination.

After exiting the space 78, the pen 10 is allowed to fall into the distribution region 56 under the force of gravity in the directly generally indicated by arrow 88. Pens 10 exiting the decontamination region 54 can be stockpiled in the distribution region 56 on another feeding cylinder 90. An optical sensor 92, for example, can optionally be utilized to monitor the number of pens 10 stockpiled to prevent the accumulation of more pens 10 in the distribution region 56 than permitted by the available space. In response to a signal from the optical sensor 92, operation of the conveyor 80 or other pen transport mechanism can be discontinued until such time the optical sensor 92 determines that additional storage space has become available. Pens 10 that have accumulated in the distribution region 56 can optionally be exposed to the decontamination agent such as the UVC light 84 while awaiting removal from the decontamination station 50, thereby promoting storage of the pen 10 in a decontaminated state.

The feeding cylinder 90 is analogous to the feeding cylinder 68 described above, and rotates to occasionally receive a decontaminated pen 10 in one, of possibly a plurality of generally U-shaped receptacles 94 formed in the feeding cylinder 90. The feeding cylinder 90 can be driven by an electric motor, can be manually rotatable, or can otherwise be controllable to rotate when removal of a pen 10 from the decontamination station 50 is desired. For example, a handle exposed to the exterior of the decontamination station 50 can be manipulated to cause rotation of the feeding cylinder 90 until the pen in the receptacle 94 is aligned with an exit 96 of the decontamination station 50, at which time the pen 10 falls under the force of gravity in the direction generally indicated by arrow 98 into a dispenser 104. The dispenser 104 can then be adjusted downward, allowing for access to, and removal of the pen 10 in a decontaminated state suitable for use in the healthcare facility.

According to alternate variants, the dispenser 104 can be operatively connected to a controller that controls operation of an electric motor or other actuation mechanism that rotates the feeding cylinder 90. When a pen 10 in a decontaminated state is desired, the dispenser 104 can be adjusted downward or otherwise manipulated to cause activation of the electric motor and, accordingly, rotation of the feeding cylinder 90.

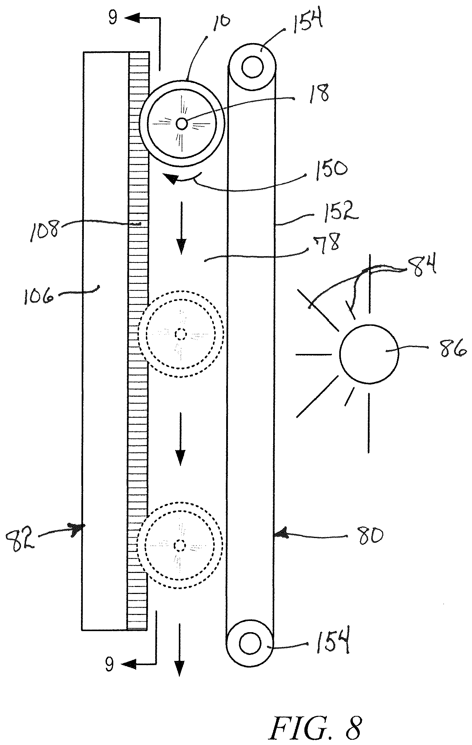

A side view of a variant of the decontamination region 54 is shown in FIG. 8 to illustrate decontamination of the pen 10, which is shown in FIG. 8 with the writing tip 18 pointed out of the paper, as it travels through the decontamination region 54. The pen 10 is shown received in the space 78 between the conveyer 80 and the stationary object 82. The stationary object can include a base plate 106 from which bristles 108 extend. The bristles 108 can be upstanding filaments, packed close together, that extend approximately 0.3175 cm (1/8 in.) to approximately 1.27 cm (1/2 in.) away from the base plate 106. As shown in FIG. 8, the pen 10 extends partially into the layer of bristles 108, and rotates in the direction generally indicated by arrow 150 as the pen 10 rolls through the decontamination region 54.

The conveyer 80 includes a belt 152 that extends continuously around a pair of pulleys 154, with a pulley 154 located adjacent to the entrance and exit of the decontamination region 54. At least one of the pulleys 154 is driven by an electric motor operated by a controller that causes rotation of the belt 152 when transportation of the pen 10 through the decontamination region 54 is desired. The belt 152 can be formed from a material that is substantially transparent to UVC light 84. For example, the belt 152 can be formed of a fine wire mesh that transmits a substantial portion, or most of the UVC light 84 emitted by the UVC bulb 86 disposed on an opposite side of the belt 152 from the pen 10. According to other variants, the belt 152 can be formed by a material that is substantially transparent to UVC light for variants that utilize UVC light as the decontamination agent.

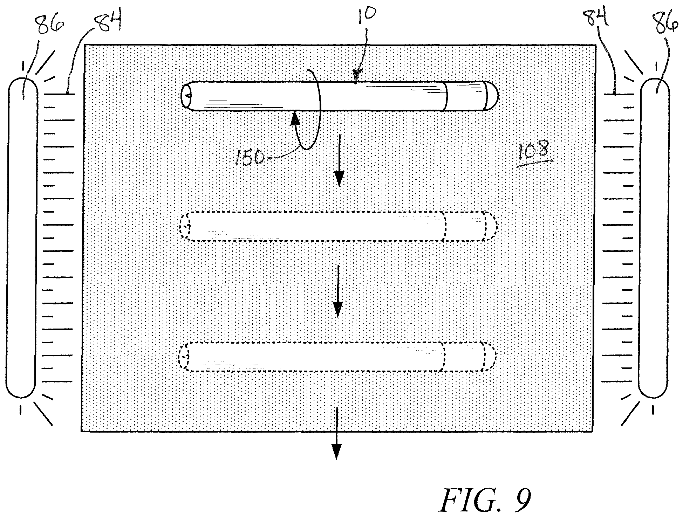

In operation, the controller can activate the electric motor that drives the pulley 154 in response to sensing the presence of a pen 10 in the hopper 58 or at the entrance of the decontamination region 54, and optionally also in response to detecting room for additional decontaminated pens 10 in the distribution region 56. Contact between the exterior periphery of the pen 10 and the belt 152 traveling about the pulleys 154 causes the pen 10 to rotate in the direction indicated generally at 150 in FIGs. 8 and 9, and roll over the bristles 108. Rotation of the pen 10 over the bristles enhances the decontamination of the pens 10, and allows all of the externally-exposed surfaces of the pens 10 to be exposed to UVC light 84 from the bulb 86 separated from the pen 10 by the belt 152. Additionally, as shown in FIGs. 7 and 9, an additional UVC bulb 86 can be arranged vertically, with a longitudinal axis in an axial direction aligned substantially parallel with the space 78 between the belt 152 and the bristles 108, adjacent to one or both ends 20, 24 of the pen 10 within the space 78. Operation of the additional, vertically-arranged bulbs 86 emits UVC light to decontaminate the exposed surfaces of the skirt 42 and internal periphery of the wall 36 at the first end 20 of the pen 10, and the exposed surfaces of the sheath 44 adjacent to the second end 24 of the pen 10. And since the horizontally-oriented UVC bulbs 86 emit UVC light imparted on the externally-exposed surface of the housing 12, Substantially all externally-exposed surfaces of the pen 10 are decontaminated.

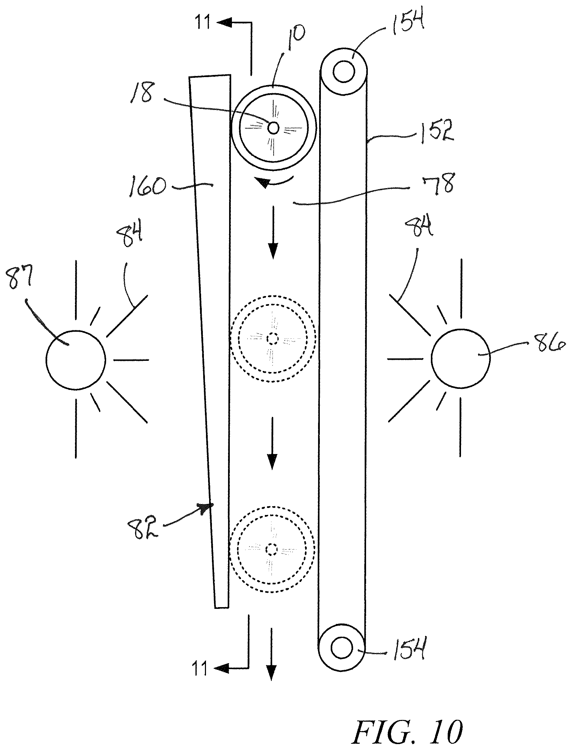

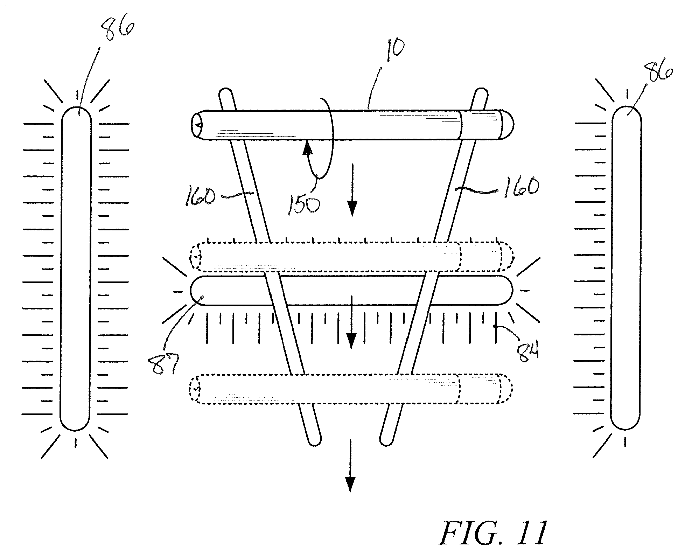

FIGs. 10 and 11 illustrate an alternate variant of the decontamination region 54. As shown in FIG. 10, the decontamination region 54 includes the belt 152 extending about the pulleys 154 to cause rotation of the pen 10 as it travels through the decontamination region 54. Light emitted from UVC bulb 86 passes through the belt 152 to reach the pen 10.

Instead of the base plate 106 and bristles 108, the stationary object 82 includes a pair of ribs 160 that are angled inward, toward each other along the path the pen 10 travels. Rotation of the belt 152 about the pulleys 154 again causes rotation of the pen 10 in the angular direction indicated by the arrow 150. However, instead of rolling over the bristles 108 as described for the variant above, the pen 10 rolls over the ribs 160. Since the ribs 160 are angled inward, toward each other at their lowermost points, no single portion of the pen 10 is concealed from UVC light 84 as the pen 10 travels through the space 78 of the decontamination region 54. Additionally, another horizontally-arranged UVC bulb 87 is separated from the pen 10 by the ribs 160, to emit UVC light onto surfaces of the housing 12 that are not in contact with, or shaded from the UVC bulb 87 by the ribs 160. But since the ribs 160 are angled as shown in FIG. 11, the surfaces of the housing 12 that are shielded by the ribs 160 will change as the pen 10 rolls along the decontamination region 54, ensuring that no single surface of the housing 12 is shielded from UVC light 84 while the pen 10 travels along the entire length of the decontamination region 54.

Although the ribs 160 are described as being angled inward, with the lower region of each rib 160 being closer to each other than the upper regions of the those ribs 160, the arrangement of the ribs 160 is not so limited. The ribs 160, of which there can be at least one, and optionally a plurality, can be configured to have any desired shape and any desired arrangement that ensures no externally-exposed surface of the housing 12 is shielded from UVC light 84 along the entire length of the decontamination region 54.

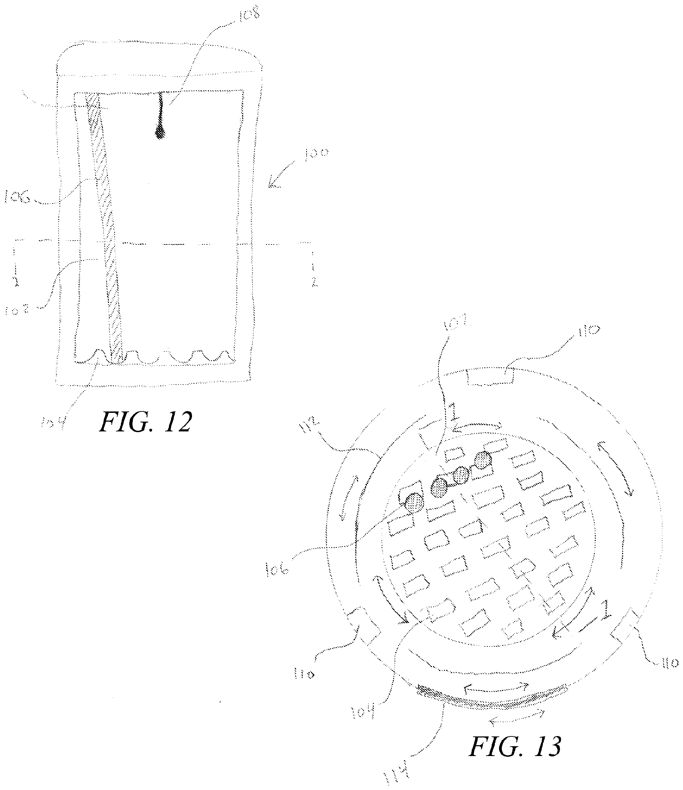

Figure 12 illustrates a cross section of an alternate variant of a disinfecting apparatus 100. The disinfecting apparatus 100 includes a disinfecting chamber 102 for placing an object to be disinfected (not shown). The disinfecting apparatus 100 may be of any size. Of particular interest in determining the size of the disinfecting apparatus 100 is the volume of the disinfecting chamber 102. There are two competing interests in determining the size of the disinfecting chamber 102. First, the intensity of the UVC light deceases as distance from the source increases according to an inverse squared relationship and the disinfecting factor of the UVC light is determined by the product of the intensity of light and the time of irradiation. Therefore, the larger a disinfecting chamber is, the longer the disinfecting process needs to be in order to reach an appropriate level of sterilization. However, it is also desirable to have a disinfecting chamber that is large enough to hold objects of various shapes and sizes. For example, a keyboard requires a disinfecting chamber that is approximately 50.8 cm (twenty inches) tall whereas a pulse oximeter would require a disinfecting chamber that is only a few cm (inches) tall. Accordingly, it may be desirable in some embodiments to have a large disinfecting chamber and in some variants to have a small disinfecting chamber. It should be noted that the size of the disinfecting apparatus is not intended to be a limiting parameter.

A floor 104 of the disinfecting chamber 102 is shown with a pyramidal shape to limit the contact area with the object placed in the disinfecting chamber 102 because such contact areas inhibit UVC light from reaching the object to disinfect it. In some variants, the floor 104 may be lined with ultraviolet c (UVC) light sources in the depressions of the pyramidal shape, thereby increasing the likelihood the object receives UVC light at or near points of contact with the floor 104. In other variants the floor 104 may be entirely UVC light sources so that even at points of contact with the object to be disinfected, the UVC light still hits the object to be disinfected. However, in variants where UVC light sources themselves act as a floor, the light sources may be subject to breaking if a heavy object is placed in the disinfecting chamber 102 or an object is dropped in to the disinfecting chamber 102. In these cases, it may be desirable to reinforce the UVC light sources. Any materials used to reinforce the UVC light sources should be substantially transparent to UVC light to permit transmission of a substantial portion (e.g., at least 60%, or at least 80%, or at least 90%, or at least 95%) of the UV light emitted by the disinfecting apparatus. One illustrative example of such a material is quartz. Another material that is substantially-transparent is polypropylene. In still other embodiments, rather than resting on the floor 104, an object to be disinfected may rest in a basket, for example, a wire-frame or mesh basket optionally formed of quartz. In such variants, UVC light emitted from UVC light sources can pass through the openings in the basket to the object where the wire frame or mesh limits the contact areas of the object that UVC light is unable to penetrate. According to other variants, the floor 104 itself can optionally be formed of fused silica or quartz, for example, and the UVC light sources arranged vertically beneath the floor, thereby separating the objects to be disinfected from the UVC light sources. For such embodiments, the floor 104 can be approximately 0.47625 cm (3/16 in.) thick to transmit approximately 80%-85% of the UVC light emitted by those UVC light sources.

Although UVC light sources are described herein as examples of the decontaminating sources, Xenon light sources or any other source of radiation that can be used to render objects materially disinfected are within the scope of the present disclosure. For instance, strobed Xenon light sources, with their operation timed to prevent operation during times when a door such as the no-touch door 114 and/or shield 112 described below are open. For any of the variants described herein not utilizing pulsed sources, operation of such sources during the disinfection process can optionally be limited to less than 30 seconds, or optionally less than 20 seconds to limit the yellowing exhibited by surfaces exposed to the sources during disinfection.

The disinfecting chamber 102 also includes a wall 106 for leaning objects to be disinfected against. Since the wall 106 represents a large contact point with the object to be disinfected, in many embodiments the wall 106 is also made of a material that is substantially-transparent to UVC light. For example, the wall 104 could be made of tubes of quartz aligned next to each other as shown in the embodiment of figure 13. The disinfecting chamber is also preferably lined with a substantially-reflective material so that UVC light waves emitted from UVC light sources can freely bounce around the disinfecting chamber, hitting the object to be disinfected at a number of angles of incidence and over the entire area of the object. According to one example, polished aluminum can be used as a substantially-reflective material. But regardless of the configuration of the disinfecting chamber 102, the disinfecting chamber 102 can optionally be sealed, optionally hermetically, to prevent air within the disinfecting chamber from venting into the ambient environment of the disinfecting apparatus 100. This seal can also prevent circulation of ambient air within the disinfecting apparatus 100. Further, the disinfecting apparatus 100 can optionally include, or be operatively connected to a vacuum source that is operable to optionally evacuate the disinfecting chamber 102.

In some variants, a hook or similar line 108, optionally formed of quartz or another material substantially transparent to UVC light, can hang from the ceiling of the disinfecting chamber 102. In such variants, an object to be disinfected may be attached to the line 108. This is particularly helpful for objects with cords, such as pulse oximeters, so that the cord may dangle freely in the disinfecting chamber and be exposed to the maximum amount of UVC light. In contrast, if the object with a cord was simply placed in the disinfecting chamber 102, the cord would "bunch" on the floor 104 with many contact points unexposed to UVC light. In some variants the end of the hook or line may include a UVC light emitting diode (LED) or similar UVC light source. In such variants, the inside of the object to be hooked may also be disinfected by the UVC light from the LED. An illustrative example of such an object is a pulse oximeter. The aperture for a finger of the pulse oximeter may be clamped to the line 102 with a UVC LED. The LED can then act as a disinfecting light source to disinfect the aperture of the pulse oximeter and the cord can hang freely in the disinfecting chamber so that it, along with the exterior surface of the pulse oximeter, can be disinfected as any other object. In other variants, rather than attaching the pulse oximeter or like object to the hook or line 108, the object may be similarly clamped to one of the tubes of the wall 104 or a standalone tube (not shown) also located in the disinfecting chamber 102. In such a variant, the pulse oximeter could be clamped on the tube as if the tube were a finger. The tube on which the pulse oximiter is to be clamped can optionally terminate short of the ceiling of the disinfection apparatus 100, allowing the pulse oximeter to be clamped onto the tube much the way it would be clamped onto a finger. Further, since the tube can be formed of quartz, UVC light can impinge on an interior surface of the pulse oximeter that would otherwise be shaded if the pulse oximeter was clamped on the end of a tube formed of a UVC opaque material.

In other variants, one or a plurality of UVC light sources (not shown) may be located at the top of the disinfecting chamber 102, adjacent to a ceiling. In such variants, the UVC light sources may hang from the ceiling. When the UVC light source hangs from the ceiling, it may be used in a manner similar to that of the UVC LED previously described. UVC light sources may also be embedded in the ceiling in a manner similar to that described with respect to the floor 104.

Figure 13 illustrates a top view of the disinfecting apparatus of the present invention. Three UVC light sources 110 are illustrated around the edge of the disinfecting chamber 102, 120 degrees from each other, such that the emitted UVC light is evenly dispersed throughout the disinfecting chamber. It should be noted that although three UVC light sources 110 are shown, a greater or fewer number, preferably at an equal angular displacement, may be used in various embodiments. A shield 112 is also shown in three parts (corresponding to the three UVC light sources) around the disinfecting chamber 102 between the UVC light sources 110 and the floor 104. In the state shown in figure 13, each of the UVC light sources 110 are open to the disinfecting chamber 102 so that UVC light can reach an object to be disinfected. In many embodiments, the UVC light sources 110 remain on as long as the disinfecting apparatus is powered. However, in other embodiments, the UVC light sources 110 may be powered off if the disinfecting apparatus 100 has not been used for a particular period of time, for example, thirty minutes.

Looking down the disinfecting chamber 102 also illustrates the pyramidal floor 104 previously described. As shown by the arrows, the floor 104 can rotate relative to the stationary UVC light sources 110 during the disinfecting procedure to expose the object to be disinfected to every UVC light source 110 at a variety of angles. According to alternate embodiments, the floor 104 can optionally be stationary, and the UVC light sources 110 rotated at least partially about the circumference of the floor 104. Regardless of whether the floor 104 or the UVC light sources 110 rotate, the angles at which UVC light emitted by the light sources 110 impinges on the object to be disinfected is adjusted during the disinfecting procedure. In this manner, substantially complete disinfection is performed about the object. Also shown are four quartz tubes creating a wall 106 for resting an object to be disinfected against as described above. As illustrated in figure 13, the wall 106 is shown as four separated tubes, offset from the center of the circular floor 104. However, in other embodiments, there may be a greater or fewer number of tubes. In still other embodiments, the tubes may aligned next to each other without any gap.

A no touch door 114 is also located along the side wall of the disinfecting chamber. One or more proximity sensors (not shown) can be used to recognize when an object to be disinfected is nearing the door to be placed inside the disinfecting chamber 102. When the sensors recognize an object has been brought into close proximity with the door 114 to be placed in the disinfecting chamber the door 114, and optionally shield parts 112, rotate such that the disinfecting chamber 102 is exposed to a user placing an object in the disinfecting chamber 102. To prevent irradiation of a user or extraneous objects, the shield parts 112 rotate to cover each of the UVC light sources 110. But again, according to alternate variants, UVC light sources 110 that are adjustable instead of installed at a fixed location can optionally move behind the shields 112. However, for the sake of brevity, the shield parts 112 are illustrated as being adjustable depending on the state of the door 114. Sensors can also optionally detect when a user's hand is removed from the disinfecting chamber 102 after depositing the object to be disinfected. Upon detecting removal of the user's hand, the door 114 and shield parts 112 rotate to again close off the disinfecting chamber 102 to the external environment and expose the UVC light sources 110 to the disinfecting chamber 102.

Although the variants described above include a disinfecting chamber 102 in which objects are to be placed, by hand, to be exposed to a disinfecting agent and rendered materially disinfected, other variants include a disinfecting chamber 102 with a floor that is located approximately at a level of a floor surface instead of a countertop on which the disinfecting apparatus 100 is placed. Such a disinfecting chamber 102 can extend upwardly a suitable height to allow hospital furniture such as an IV stand on which IV bags are suspended, to be rolled into the disinfecting chamber 102 without having to be elevated above more than a molding exposed while the door is open. Such variants may include a door that closes all the way to the floor surface on which the disinfecting apparatus rests 100, preventing the UVC light from exiting the disinfecting apparatus 100 during operation. In use, the IV stand or other wheeled object to be rendered materially disinfected can be rolled over a mat or otherwise wiped down with a disinfectant when the IV stand is rolled into or out of the disinfecting apparatus 100. An infusion pump or other object supported by the IV stand can optionally remain in place during operation of the disinfecting apparatus 100, also rendering the exposed surfaces of those objects materially disinfected. Such usage would not expose every single surface of the IV stand, infusion pump and/or other object to the UVC light. However, the exposed surfaces of those objects that operators will come into contact with during typical usage are rendered materially disinfected.

Yet other variants of the disinfecting apparatus 100 can be configured to positively receive and retain the object to be rendered materially disinfected. For example, the disinfecting apparatus 100 can be configured as a stand with one or a plurality of disinfecting receivers. Each receiver can include one or a plurality of UVC light sources, optionally arranged to extend about the region in which the object is to be received. To accommodate objects of different sizes and shapes, a collar can be clamped to each of those different objects and coupled to a receiver. Thus, the collars are all configured to be compatible with the receiver, but also to be clamped to different objects, thereby ensuring the objects are properly held in place adjacent to the UVC light sources to render those objects materially disinfected. According to alternate variants, the collars themselves can be provided with UVC light sources such that the collars can be installed on the different objects and the UVC light sources energized to render the objects materially disinfected at a place of use. Regardless of the configuration, a curtain or other flexible blanket that is opaque to UVC light can be draped over the collars, or at least extends downwardly from the collars to interfere with the transmission of UVC light into the ambient environment.

To the extent that the term "includes" is used in either the detailed description or the claims, such term is intended to be inclusive in a manner similar to the term "comprising" as "comprising" is interpreted when employed as a transitional word in a claim.

## Claims

1. An apparatus (100) for disinfecting objects of various shapes and sizes, the apparatus comprising:
a housing enclosing a disinfecting chamber (102) in which objects are to be placed;
ultraviolet light sources (110) angularly displaced around the edge of the disinfecting chamber (102) to emit ultraviolet light into the disinfecting chamber (102) to be imparted on the object in the disinfecting chamber (102) for deactivating at least a portion of the biologically-active contaminant present on the object;
a liner provided to the disinfecting chamber (102) and comprising a reflective element so that the ultraviolet light is reflected around the chamber (102) to provide maximum incidence with the object;
the apparatus being **characterized by**
a wall (106) within the disinfecting chamber (102), the wall being formed at least in part from a material that is substantially-transparent to ultraviolet light against which objects are to be leaned, wherein the ultraviolet light is transmitted through the material to reach a portion of the object contacting the wall (106); and
a floor (104) of the disinfecting chamber (102) with a pyramidal shape with depressions to limit a contact area
with the object in the disinfecting chamber (102), wherein the floor (104) rotates within the disinfecting chamber (102) during a disinfecting procedure relative to the ultraviolet light source (110) to expose the object to be disinfected to every ultraviolet light source (110) at a variety of angles and further promote maximum incidence between the object and
ultraviolet light source (110).

2. The apparatus (100) of claim 1 further comprising a door (114) located along the side wall of the disinfecting chamber that allows users to insert and remove objects to and from the disinfecting chamber (102) without transferring biologically- active contaminants from their hands to the chamber (102); and
a proximity sensor that detects when the object to be disinfected is nearing the door (114) to be placed inside the disinfecting chamber (102) and causes the door (114) to rotate such that it opens to grant access to the disinfecting chamber (102).

3. The apparatus (100) of claim 2 further comprising a plurality of shields (112) that rotate to cover each of the UVC light sources (110) to prevent the ultraviolet light emitted by the ultraviolet light sources (110) from escaping the disinfection chamber (102) while the door (114) is open.

4. The apparatus (100) of claim 3 further comprising a sensor that senses the object has been placed within the disinfecting chamber (102) and transmits a signal that causes the door (114) to rotate closed along with the shield and the ultraviolet light source (110) to be activated.

5. The apparatus (100) of claim 1 further comprising a hanging device that extends downward from an upper surface of the disinfecting chamber (102) from which the object is to be hung.

6. The apparatus (100) of claim 1, wherein the wall (104) is made of tubes of quartz aligned next to each other.

## Patentansprüche

1. Vorrichtung (100) zum Desinfizieren von Gegenständen verschiedener Formen und Größen, wobei die Vorrichtung umfasst:
ein Gehäuse, das eine Desinfektionskammer (102) umschließt, in der Gegenstände platziert werden können; ultraviolette Lichtquellen (110), die winklig um den Rand der Desinfektionskammer (102) herum angeordnet sind, um ultraviolettes Licht in die Desinfektionskammer (102) zu emittieren, das auf den Gegenstand in der Desinfektionskammer (102) einwirken soll, um zumindest einen Teil der auf dem Gegenstand vorhandenen biologisch aktiven Verunreinigung zu deaktivieren;
eine an der Desinfektionskammer (102) vorgesehene Auskleidung mit einem reflektierenden Element, so dass das ultraviolette Licht um die Desinfektionskammer (102) herum reflektiert wird, um ein maximales Auftreffen auf den Gegenstand zu ermöglichen; wobei die Vorrichtung **gekennzeichnet ist durch** eine Wand (106) innerhalb der Desinfektionskammer (102), wobei die Wand zumindest teilweise aus einem Material gebildet wird, das für ultraviolettes Licht, gegen das Gegenstände angelehnt werden sollen, im Wesentlichen durchlässig ist, wobei das ultraviolette Licht durch das Material hindurch übertragen wird, um einen Teil des Gegenstandes zu erreichen, der die Wand (106) berührt; und
einen Boden (104) der Desinfektionskammer (102) in Pyramidenform mit Vertiefungen zur Begrenzung einer Kontaktfläche mit dem Gegenstand in der Desinfektionskammer (102), wobei sich der Boden (104) in der Desinfektionskammer (102) während eines Desinfektionsvorgangs relativ zu der ultravioletten Lichtquelle (110) dreht, um den zu desinfizierenden Gegenstand jeder ultravioletten Lichtquelle (110) einer Vielzahl von Winkeln auszusetzen und weiterhin einen maximalen Einfall zwischen dem Gegenstand und der ultravioletten Lichtquelle (110) zu fördern.

2. Die Vorrichtung (100) nach Anspruch 1 umfasst ferner eine Tür (114), die sich entlang der Seitenwand der Desinfektionskammer befindet und es Benutzern ermöglicht, Gegenstände in die Desinfektionskammer (102) einzuführen und aus ihr zu entfernen, ohne biologisch aktive Verunreinigungen von ihren Händen in die Kammer (102) zu übertragen; und
einen Näherungssensor, der erkennt, wenn sich der zu desinfizierende Gegenstand der Tür (114) nähert, um in der Desinfektionskammer (102) platziert zu werden, und der bewirkt, dass sich die Tür (114) so dreht, dass sie sich öffnet, um Zugang zur Desinfektionskammer (102) zu gewähren.

3. Die Vorrichtung (100) nach Anspruch 2 umfasst ferner eine Vielzahl von Abschirmungen (112), die sich drehen, um jede der UVC-Lichtquellen (110) abzudecken, um zu verhindern, dass das von den UVC-Lichtquellen emittierte ultraviolette Licht aus der Desinfektionskammer (102) entweicht, während die Tür (114) geöffnet ist.

4. Die Vorrichtung (100) nach Anspruch 3 umfasst ferner einen Sensor, der erkennt, dass der Gegenstand in die Desinfektionskammer (102) eingebracht wurde, und ein Signal sendet, das bewirkt, dass die Tür (114) zusammen mit der Abschirmung geschlossen wird und die ultraviolette Lichtquelle (110) aktiviert wird.

5. Die Vorrichtung (100) nach Anspruch 1 umfasst ferner eine Aufhängevorrichtung, die sich von einer oberen Fläche der Desinfektionskammer (102), an der der Gegenstand aufgehängt werden soll, nach unten erstreckt.

6. Vorrichtung (100) nach Anspruch 1, wobei die Wand (104) aus nebeneinander angeordneten Quarzröhren besteht.

## Revendications

1. Appareil (100) pour désinfecter des objets de différentes formes et tailles, l'appareil comprenant :
un boîtier renfermant une chambre de désinfection (102) dans laquelle des objets doivent être placés ;
des sources de lumière ultraviolette (110) déplacées angulairement autour du bord de la chambre de désinfection (102) pour émettre de la lumière ultraviolette dans la chambre de désinfection (102) pour être imprimées sur l'objet dans la chambre de désinfection (102) pour désactiver au moins une partie du contaminant biologiquement actif présent sur l'objet ;
un revêtement fourni à la chambre de désinfection (102) et comprenant un élément réfléchissant de sorte que la lumière ultraviolette est réfléchie autour de la chambre (102) pour fournir une incidence maximale avec l'objet ;
l'appareil étant **caractérisé par** :
une paroi (106) à l'intérieur de la chambre de désinfection (102), la paroi étant formée au moins en partie d'un matériau qui est sensiblement transparent à la lumière ultraviolette contre laquelle les objets doivent être appuyés, dans lequel la lumière ultraviolette est transmise à travers le matériau pour atteindre une partie de l'objet en contact avec la paroi (106) ; et
un plancher (104) de la chambre de désinfection (102) de forme pyramidale avec des dépressions pour limiter une zone de contact avec l'objet dans la chambre de désinfection (102), dans lequel le plancher (104) tourne à l'intérieur de la chambre de désinfection (102) au cours d'une procédure de désinfection par rapport à la source de lumière ultraviolette (110) pour exposer l'objet à désinfecter à chaque source de lumière ultraviolette (110) selon une variété d'angles et favoriser davantage une incidence maximale entre l'objet et la source de lumière ultraviolette (110).

2. Appareil (100) selon la revendication 1, comprenant en outre une porte (114) située le long de la paroi latérale de la chambre de désinfection qui permet aux utilisateurs d'insérer et d'enlever des objets vers et depuis la chambre de désinfection (102) sans transférer de contaminants biologiquement actifs de leurs mains vers la chambre (102) ; et
un capteur de proximité qui détecte le moment où l'objet à désinfecter s'approche de la porte (114) à placer à l'intérieur de la chambre de désinfection (102) et provoque la rotation de la porte (114) de sorte qu'elle s'ouvre pour donner accès à la chambre de désinfection (102).

3. Appareil (100) selon la revendication 2, comprenant en outre une pluralité de blindages (112) qui tournent pour couvrir chacune des sources de lumière UVC (110) pour empêcher la lumière ultraviolette émise par les sources de lumière ultraviolette (110) de s'échapper de la chambre de désinfection (102) lorsque la porte (114) est ouverte.

4. Appareil (100) selon la revendication 3, comprenant en outre un capteur qui détecte que
l'objet a été placé à l'intérieur de la chambre de désinfection (102) et transmet un signal qui amène la porte (114) à tourner en fermeture avec l'écran et la source de lumière ultraviolette (110) à être activée.

5. Appareil (100) selon la revendication 1, comprenant en outre un dispositif de suspension qui s'étend vers le bas à partir d'une surface supérieure de la chambre de désinfection (102) à partir de laquelle l'objet doit être suspendu.

6. Appareil (100) selon la revendication 1, dans lequel la paroi (104) est constituée de tubes de quartz alignés les uns à côté des autres.
